# EUROPÄISCHE PATENTSCHRIFT

(11) **EP 1 259 126 B1**
(45) Veröffentlichungstag und Bekanntmachung des Hinweises auf die Patenterteilung: **11.05.2005**
(21) Anmeldenummer: 00993845.7
(22) Anmeldetag: 27.12.2000
(51) Int. Cl.: A23L 1/30, A23L 2/00, A61K 35/78

(54) **STÄRKENDES NATURHEILGETRÄNK**
STRENGTHENING NATURAL HEALING DRINK
BOISSON NATURELLE FORTIFIANTE

(30) Priorität: 02.03.2000 CZ 200010457 U
(43) Veröffentlichungstag der Anmeldung: 27.11.2002
(73) Patentinhaber: Belak, Jiri, 403 23 Velké Brezno (CZ)
(72) Erfinder: Belak, Jiri, 403 23 Velké Brezno (CZ)
(74) Vertreter: Jeck, Anton, Dipl.-Ing.
(86) Internationale Anmeldenummer: PCT/CZ2000/000092
(87) Internationale Veröffentlichungsnummer: WO 2001/064058

(56) Entgegenhaltungen:
- CH-A- 685 373
- DE-A- 19 838 462
- DATABASE WPI Section Ch, Week 199536 Derwent Publications Ltd., London, GB; Class D13, AN 1995-273879 XP002165374 & RU 2 027 383 C (UKR DRINKS RES INST), 27. Januar 1995 (1995-01-27)

## Beschreibung

### Technikbereich

Die Erfindung betrifft ein alkoholfreies Getränk auf Basis gesunder Naturstoffe aus Pflanzen und Wasser.

### Gegenwärtiger Stand der Technik

Die bisher bekannten alkoholfreien Getränke auf Basis mit Kohlendioxyd gesättigten Wasser und mit nur einem kleinen Mengenanteil an Naturobstsäften werden zur Geschmacksund Aussehensunterstreichung nachgefärbt, mit Aromastoffen abgeschmeck und mit Zucker oder künstlichen Einkomponentensüsstoffen gesüsst.

Solche Getränke entsprechen nicht den gegenwärtigen Heilungsbemühungen des Organismus durch gusunde Ernährung auf Basis Naturprodukte mit Einschränkung der Zuckerkonsumierung und chemischer Zusatzstoffe, wie z.B. Aromastoffe, Farbstoffe und Süsstoffe.

### Gegenstand der Erfindung

Das alkoholfreie stärkende Naturheilgetränk laut Gebrauchsmuster auf Basis gesunder Naturstoffe aus Pflanzen und Wasser, ohne Zucker und ohne chemische Aroma-und Farbstoffe besteht aus 5% Absudmasse aus 0,05g bis 15g echten Kamillenblüten in 50 ml Trinkwasser, 0,01 % bis 0,75% Galaktosemasse, 0,01 % bis 0,75% Arabinosemasse, 0,01 % bis 0,75% Xylosemasse, 0,01% bis 0,75% Ramnosemasse, 5% Absudmasse aus 0,05g bis 15g Brennesselblatt in 50 ml Trinkwasser, 0,015% bis 1,5% Myristosesäuremasse, 0,01% bis 1% Stearsäuremasse, 0,015% bis 1,5% Palmitsäuremasse, 0,125% bis 12,5% Masse pflanzlicher Eiweisstoffe, 5% Absudmasse aus 0,05g bis 15g Lindenblüte in 50 ml Trikwasser, 0,15% bis 15% Linolsäuremasse, 5% Absudmasse aus 0,05g bis 15g Kraut vom gewöhnlichen Odermennig in 50 ml Trikwasser, 0,125% bis 12,5% Linolensäuremasse, 5% Absudmasse aus 0,05g bis 15g Tee in 50 ml Trinkwasser, 0,025% bis 2,5% Ölsäuremasse und in 100% Trinkwassermasse.

### Ausführungsbeispiel des Gebrauchsmusters

Getränkerezeptur nach technischer Lösung ("Belarex")

| | | |
|---|---|---|
| Absud aus 0,5g echten Kamillenblüten in 50 ml Trinkwasser | 5 | % Masse |
| Galaktose | 0,1 | % Masse |
| Arabinose | 0,1 | % Masse |
| Xylose | 0,1 | % Masse |
| Ramnose | 0,1 | % Masse |
| Absud aus 0,5g Brennesselblatt in 50 ml Trinkwasser | 5 | % Masse |
| Myristsäure | 0,15 | % Masse |
| Stearsäure | 0,1 | % Masse |
| Palmitsäure | 0,15 | % Masse |
| Pflanzliche Eiweisstoffe | 1,25 | % Masse |
| Absud aus 0,5g Lindenblüte in 50 ml Trinkwasser | 5 | % Masse |
| Linolsäure | 1,5 | % Masse |
| Absud aus 0,5g Kraut vom gewöhnlichen Odermennig in | | |
| 50 ml Trinkwasser | 5 | % Masse |
| Linolensäure | 1,25 | % Masse |
| Absud aus 0,5g Indischen oder Chinesischen Tee in 50 ml | | |
| Trinkwasser | 5 | % Masse |
| Ölsäure | 0,25 | % Masse |
| Trinkwasser bis | 100 | % Masse |

Die oben angeführten Stoffe werden in beliebiger Reihenfolge gemischt.

### Industrienutzung

Das alkoholfreie stärkende Naturheilgetränk aus gesunden Naturstoffen aus Pflanzen und Trinkwasser, ohne Zucker und ohne chemischen Aroma-und Farbstoffen entspricht den hohen Ansprüchen einer gesunden Ernährung.

## Patentansprüche

1. Das stärkende Naturheilgetränk **dadurch gekennzeichnet**, **dass** es aus 5% Absudmasse aus 0,05g bis 15g echten Kamillenblüten in 50 ml Trinkwasser, 0,01% bis 0,75% Galaktosemasse, 0.01% bis 0,75% Arabinosemasse, 0,01 % bis 0,75% Xylosemasse, 0,01% bis 0,75% Ramnosemasse, 5% Absudmasse aus 0,05g bis 15g Brennesselblatt in 50 ml Trinkwasser, 0,015% bis 1,5% Myristsäuremasse, 0,01% bis 1% Stearsäuremasse, 0,015% bis 1,5% Palmitsäuremasse, 0,125% bis 12,5% Masse pflanzlicher Eiweisstoffe, 5% Absudmasse aus 0,05g bis 15g Lindenblüte in 50 ml Trikwasser, 0,15% bis 15% Linolsäuremasse, 5% Absudmasse aus 0,05g bis 15g Kraut vom gewöhnlichen Odermennig in 50 ml Trikwasser, 0,125% bis 12,5% Linolensäuremasse, 5% Absudmasse aus 0,05g bis 15g Tee in 50 ml Trinkwasser, 0,025% bis 2,5% Ölsäuremasse und in bis 100% Trinkwassermasse besteht.

## Claims

1. The invigorating natural health drink, **characterised in that** it comprises 5 % extract composition formed from between 0.05 g and 15 g genuine camomile blossom in 50 ml drinking water, between 0.01 % and 0.75 % lactose composition, between 0.01 % and 0.75 % arabinose composition, between 0.01 % and 0.75 % xylose composition, between 0.01 % and 0. 75 % ramnose composition, 5 % extract composition formed from between 0.05 g and 15 g stinging nettle leaf in 50 ml drinking water, between 0.015 % and 1.5 % myristic acid composition, between 0.01 % and 1 % stearic acid composition, between 0.015 % and 1.5 % palmitic acid composition, between 0.125 % and 12.5 % composition of vegetable proteins, 5 % extract composition formed from between 0.05 % and 15 % lime tree blossom in 50 ml drinking water, between 0.15 % and 15 % linoleic acid composition, 5 % extract composition formed from between 0.05 g and 15 g herb from common agrimony in 50 ml drinking water, between 0.125 % and 12.5 % linoleic acid composition, 5 % extract composition formed from between 0.05 g and 15 g tea in 50 ml drinking water, between 0.025 % and 2.5 % oleic acid composition, and drinking water composition to make up to 100 %.

## Revendications

1. Boisson médicinale naturelle fortifiante **caractérisée en ce qu'**elle est constituée de 5% en masse d'une décoction de 0,05 g à 15 g de véritables fleurs de camomille dans 50 ml d'eau potable, de 0,01 % à 0,75 % en masse de galactose, de 0,01 % à 0,75 % en masse d'arabinose, de 0,01 % à 0,75 % en masse de xylose, de 0,01 % à 0,75 % en masse de ramnose, de 5 % en masse d'une décoction de 0,05 g à 15 g de feuilles d'ortie dans 50 ml d'eau potable, de 0,015 % à 1,5 % en masse d'acide myristique, de 0,01 % à 1 % en masse d'acide stéarique, de 0,015 % à 1,5 % en masse d'acide palmitique, de 0,125 % à 12,5 % en masse d'albumine végétale, de 5 % en masse d'une décoction de 0,05 g à 15 g de fleurs de tilleul dans 50 ml d'eau potable, de 0,15 % à 15 % en masse d'acide linoléique, de 5% en masse d'une décoction de 0,05 g à 15 g d'herbe d'aigremoine commune dans 50 ml d'eau potable, de 0,125 % à 12,5 % en masse d'acide linoléique, de 5% en masse d'une décoction de 0,05 g à 15 g de thé dans 50 ml d'eau potable, de 0,025 % à 2,5 % en masse d'acide oléique et dans un maximum de 100 % en masse d'eau potable.
